# EUROPÄISCHE PATENTANMELDUNG

(11) **EP 2 368 535 A1**
(43) Veröffentlichungstag der Anmeldung: **28.09.2011**
(21) Anmeldenummer: 10157241.0
(22) Anmeldetag: 22.03.2010
(51) Int. Cl.: A61G 12/00

(54) **Personenüberwachungssystem**

(71) Anmelder: Polat, Candemir, 4106 Therwil (CH)
(72) Erfinder: Polat, Candemir, 4106 Therwil (CH)
(74) Vertreter: Braun, André jr.

(57) **Zusammenfassung**

Die Erfindung betrifft ein Personenüberwachungssystem (1) und ein entsprechendes Verfahren zum Überwachen von Körperbewegungen einer Person in einem Bett (10), wobei Berührungen einer Seitensicherung (100) des Betts durch die Person mittels druckempfindlichen Detektionsmitteln (102) erfasst werden und ein entsprechendes Überwachungssignal generiert und an ein Empfangsmodul (157) des Personenüberwachungssystems (1) übertragen wird, wobei die Seitensicherung (100) horizontal und vertikal beweglich gelagert ist, wobei mittels druckempfindlichem Detektionsmittel ein auf die Seitensicherung ausgeführter Druck als Druckvektor (F) erfasst und entsprechende Druckparameter generiert werden, wobei mittels eines Weichenmoduls (153) des Personenüberwachungssystems (1) die generierten Druckparameter mit in einer Lookup-Table (151) des Personenüberwachungssystems (1) speicherbaren Druckparametern verglichen werden, wobei den gespeicherten Druckparametern entsprechende Überwachungsmuster zugeordnet sind, wobei mittels bestimmbaren Vergleichskriterien des Weichenmoduls (153) beim Vergleich der Druckparameter ein entsprechendes Überwachungssignal generiert wird, wobei das Überwachungssignal mittels eines Interfacemoduls (155) des Personenüberwachungssystems (1) zum Übertragen von Signalen an das Empfangsmodul (157) übertragen wird.

## Beschreibung

Personenüberwachungssysteme zum Überwachen von Körperbewegungen einer Person in einem Bett und zum Herbeirufen von Personal, wie etwa im Spitalbereich, sind bekannt.

In der Patentschrift US 4067005 A1 wird ein Alarmsystem für eine pflegebedürftige Person in einem Spitalbett beschrieben, wobei das Spitalbett elektrische Schalter aufweist, welche auf der oberen Kante des seitlichen Bettgeländers und am Fussgeländer angebracht sind, wobei bei Krafteinwirkung durch die Person auf einen Schalter eine Alarmeinrichtung aktiviert wird. Zudem sind Mittel vorhanden, mittels welchen das Spitalbett selektiv beleuchtbar ist. In einer ersten Konfiguration werden die Schalter dazu verwendet, um bei leichtem Druck bzw. Krafteinwirkung anzusprechen. In einer weiteren Konfiguration sprechen die Schalter erst bei starker Krafteinwirkung oder Druck an, bspw. wenn ein Patient aus dem Bett steigen oder über die Geländer klettern will, was bei einem nachfolgenden Sturz aus dem Bett zu Verletzungen führen könnte.

Einer der Nachteile der Erfindung besteht darin, dass die elektrischen Schalter derart angeordnet sind, dass die pflegebedürftige Person über das Geländer steigen muss, damit die Alarmeinrichtung aktiviert wird. Die Krafteinwirkung hat demnach vorzugsweise in vertikaler Richtung auf die Schalter bzw. Schaltbalken zu erfolgen. Dadurch wird erst sehr spät, nämlich erst wenn die Person beim Verlassen des Bettes ist, ein Alarm ausgelöst.

Das Dokument US 3781843 A1 offenbart eine Einrichtung um Pflegepersonal zu alarmieren, wenn ein Patient aus seinem Bett steigen will, wobei die Einrichtung Mittel mit einem mit Fluid gefüllten Durchgang aufweist, welche an ein Seitengeländer oder das Fussgeländer des Betts angeordnet werden können. Die Mittel sind mit Umwandlern verbunden, mittels welchen der Alarm aktivierbar ist, wenn eine bestimmte Menge an Fluid verdrängt worden ist.

Einer der Nachteile der Erfindung besteht darin, dass zur Umwandlung einer Bewegung der Person in ein Alarmsignal ein Fluid verwendet wird. Bei Beschädigung des Detektionsmittels bzw. Umwandlers kann das Fluid austreten, was im Pflege- und Medizinalbereich ungeeignet ist. Zudem ist die Wartung umständlich. Des Weiteren ist der Detektionsbereich des Umwandlers nur schwer kalibrierbar.

Es ist daher Aufgabe der vorliegenden Erfindung, ein System und ein entsprechendes Verfahren bereitzustellen, welches die Nachteile des Stands der Technik nicht aufweist. Insbesondere soll das System fehlerresistent sein. Eine weitere Aufgabe besteht darin, dass das System flexibel konfigurierbar sein soll, bspw. indem es an unterschiedliche Pflegetypen angepasst werden kann. Zudem soll das System kostengünstig realisierbar sein.

Insbesondere wird die Aufgabe der Erfindung gelöst durch ein Personenüberwachungssystem zum Überwachen von Körperbewegungen einer Person in einem Bett, wobei das Bett eine Seitensicherung an einer Seite des Betts umfasst, dass die Seitensicherung mit druckempfindlichen Detektionsmitteln gekoppelt, wobei Körperbewegungen der Person bei Berührung der Seitensicherung erfassbar und mittels eines Interfacemoduls des Personenüberwachungssystems zum Übertragen eines entsprechenden Überwachungssignals an ein Empfangsmodul übertragbar sind, wobei die Seitensicherung horizontal und vertikal beweglich gelagert ist, wobei mittels druckempfindlichem Detektionsmittel ein auf die Seitensicherung ausgeführter Druck als Druckvektor erfassbar ist, das Personenüberwachungssystem eine Lookup-Table zum Speichern von Druckparametern umfasst, wobei jedem Druckparameter entsprechende Überwachungsmuster zugeordnet sind, mittels eines Weichenmoduls des Personenüberwachungssystems durch Vergleich von Druckparametern des erfassten Druckvektors mit Druckparametern der Lookup-Table ein entsprechendes Überwachungsmuster aus der Lookup-Table auslesbar ist, basierend auf welchem ein entsprechendes Überwachungssignal generierbar ist und mittels Interfacemodul an das Empfangsmodul übertragbar ist.

Einer der Vorteile der Erfindung besteht darin, dass Bewegungen der zu überwachenden Person bereits dann detektierbar sind, wenn die sich noch im Bett befindliche Person gegen die Seitensicherung, welche bspw. als Bettgeländer ausgebildet ist, eine Kraft bzw. einen Druck ausübt. Dies kann bspw. durch eine unbeabsichtigte Verlagerung der Person geschehen. Diese Kraftausübung wird als seitliche Bewegungskomponente mittels des Personenüberwachungssystem erfasst und ein entsprechender Alarm generiert, welcher an das Pflegepersonal übermittelt wird.

Beabsichtigt die Person sogar, über das Geländer zu steigen, so wird eine im Wesentlichen vertikale Kraftausübung auf das Geländer bzw. die Seitensicherung ausgeübt und detektiert und ein entsprechender Alarm generiert. Hilfe kann somit gestützt auf bewusste wie auch unbewusste Bewegungen der bettlägerigen Person herbeigerufen werden. Die Bewegungskomponenten in horizontaler und vertikaler Richtung können einzeln oder in Kombination detektiert, ausgewertet und zum Generieren eines entsprechenden Alarm- bzw. Überwachungssignals genutzt werden.

In einer Ausführungsvariante des Personenüberwachungssystems sind dem bzw. jedem Druckvektor die Druckparameter Druckdauer und/oder Druckrichtung und/oder Druckstärke zuordenbar und in der Lookup-Table speicherbar sind. Einer der Vorteile der Erfindung besteht darin, dass basierend auf einer Vielzahl von gespeicherten Druckparametern eine Vielzahl von Überwachungsmustern definierbar ist. So können Unterscheidungen zwischen Druckstärke, -richtung und -dauer in verschiedenen Kombinationen oder alleine genutzt werden, um entsprechende Überwachungsmuster zu selektieren. Insbesondere können Überwachungsmuster für bewusste, aber auch für unbewusste Bewegungen festgelegt werden. Bewusste Überwachungsmuster können bspw. Morse-ähnliche Betätigungen der Seitensicherung sein, etwa ein Betätigungs- bzw. Bewegungsmuster für Hunger oder für Durst oder für Schmerzen.

In einer anderen Ausführungsvariante des Personenüberwachungssystems weist jede Seitensicherung an deren mehrere druckempfindliche Detektionsmittel auf. Einer der Vorteile der Erfindung besteht darin, dass bspw. sowohl die Druck- oder Kraftwirkung einer Person mit den Armen sowie gleichzeitig mit den Beinen gegen unterschiedliche Holmen der Seitensicherung detektierbar und entsprechende Überwachungssignale übertragbar sind. Überlagernde Druckvektoren können so detektiert werden.

Im Folgenden wird die Erfindung anhand der in den Zeichnungen dargestellten Ausführungsbeispiele näher erläutert. Hierbei gehen aus den Zeichnungen und ihrer Beschreibung weitere wesentliche Merkmale und Vorteile der Erfindung hervor.
Fig. 1 zeigt eine schematische perspektivische Ansicht des erfindungsgemässen Personenüberwachungssystems;
Fig. 2 zeigt ein Detail des Personenüberwachungssystems;
Fig. 3 zeigt ein anderes Detail des Personenüberwachungssystems;
Fig. 4 zeigt ein weiteres Detail des Personenüberwachungssystems;
Fig. 5 zeigt ein anderes Detail des Personenüberwachungssystems;
Fig. 6 und Fig. 7 zeigen eine Ausführungsvariante des Personenüberwachungssystems;
Fig. 8 zeigt ein prinzipielles Blockschaltbild des erfindungsgemässen Personenüberwachungssystems;
Fig. 9 zeigt eine vereinfacht dargestellt Führungsschiene des Personenüberwachungssystems mit einem Führungselement und
Fig. 10a bis 10d zeigen die beweglich gelagerte Seitensicherung des erfindungsgemässen Personenüberwachungssystems in einer anderen Ausführungsvariante.

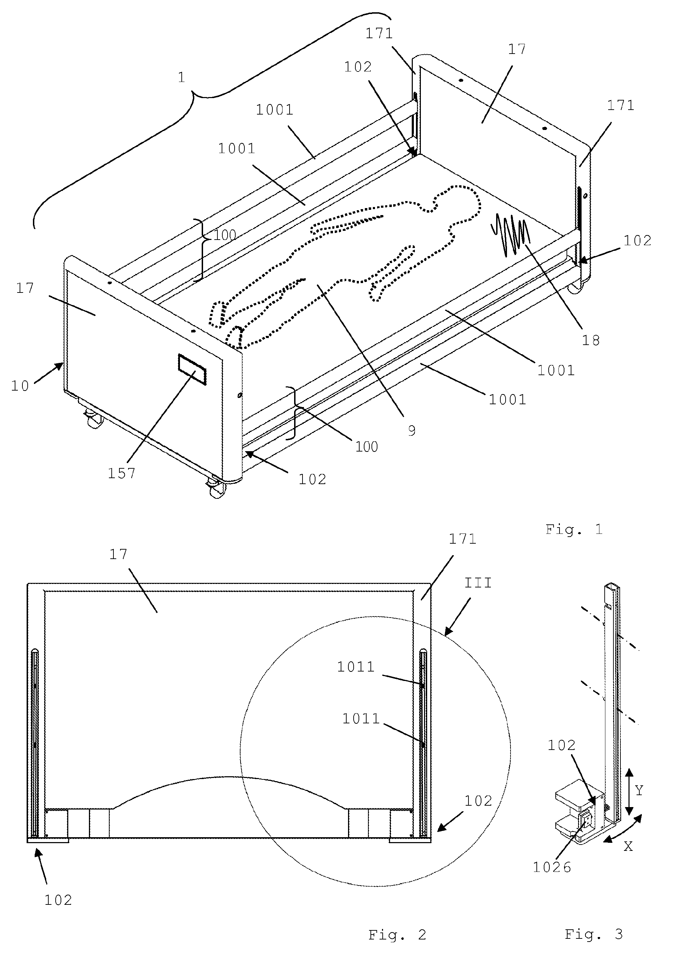

Figur 1 illustriert das erfindungsgemässe Personenüberwachungssystem 1 zum Überwachen von Körperbewegungen einer Person in einem Bett 10, wobei das Bett eine Seitensicherung 100 an einer Seite des Betts umfasst. Die Seitensicherung ist mit druckempfindlichen Detektionsmitteln 102 gekoppelt, wobei Körperbewegungen der Person bei Berührung der Seitensicherung erfassbar und mittels eines Interfacemoduls 155 des Personenüberwachungssystems zum Übertragen eines entsprechenden Überwachungssignals an ein Empfangsmodul 157 übertragen werden. Die Seitensicherung 100 ist horizontal und vertikal beweglich gelagert, wobei mittels druckempfindlichem Detektionsmittel ein auf die Seitensicherung ausgeführter Druck als Druckvektor F erfassbar ist. Horizontal beweglich bedeutet, dass bei Druckrichtung seitlich auf die Seitensicherung letztere sich auch seitlich in einem bestimmten Mass, vorzugsweise wenige Millimeter, bewegen kann. Vertikal beweglich bedeutet, dass sich bei Druckrichtung senkrecht auf den oder die Holme der Seitensicherung letztere sich um ein bestimmtes Mass, vorzugsweise wenige Millimeter bewegen kann.

Das Personenüberwachungssystem 1 umfasst eine Lookup-Table 151 zum Speichern von Druckparametern, wobei jedem Druckparameter entsprechende Überwachungsmuster zugeordnet sind.

Mittels eines Weichenmoduls 153 des Personenüberwachungssystems 1 werden die Druckparametern des erfassten Druckvektors mit Druckparametern der Lookup-Table 151 verglichen und ein entsprechendes Überwachungsmuster aus der Lookup-Table ausgelesen.

Basierend auf dem entsprechenden Überwachungsmuster wird mittels des Weichenmoduls ein Überwachungssignal generiert. Dieses wird mittels Interfacemodul 155 an das Empfangsmodul 157 übertragbar ist.

Figur 3 illustriert in einer Detailansicht von Figur 2 die mit Bezugszeichen 102 im Bereich eines Bett- oder Eckpfostens 171 angeordneten druckempfindlichen Detektionsmittel 1026. Die Kraft- bzw. Druckwirkung auf das Detektionsmittel 1026 wird von den in Figur 1 ersichtlichen Längsholmen 1001 auf die Führungsschiene 101, welche an den Betteilen 17 angeordnet sind, und mittels der Führungsschiene auf das Kopplungsteil 105 übertragen. Mittels am Kopplungsteil gekoppelte Detektionsmitteln, bspw. Schalter 1026 oder Dehnmessstreifen 1027, wird der in eine geringfügige X-und/oder Y-Bewegungskomponente umgewandelte Druckvektor erfasst und ein entsprechendes Sensorsignal generiert.

Zur Erfassung des Druckvektors F ist mindestens ein elektrischer Schalter zum Erfassen von Druckdauer und/oder Druckrichtung und/oder Druckstärke vorhanden. Vorzugsweise werden elektrische Mikroschalter verwendet. Der Schaltkontakt wird vorzugsweise über einen mit einer Rückstossfeder versehenen Stift von der Führungsschiene aus oder direkt von dieser betätigt.

Die Seitensicherung 100 ist in einem Führungssystem 101 höhenverstellbar und/oder schwenkbar gelagert ist, wobei das Führungssystem mit den Detektionsmitteln 102 gekoppelt. Die Seitensicherung kann bspw. zur Pflege der bettlägerigen Person bspw. unter den Lattenrost weggeschwenkt werden. Die Seitensicherung kann auch in der Höhe tief positioniert werden, dass die Pflege erleichtert wird. So kann die Seitensicherung 100 auf einer Bettseite bspw. auf die Höhe der Matratze 18 heruntergeschoben werden. Die Seitensicherung wird dann im Betrieb dann ein Überwachungssignal übertragen, wenn die Person über der Bettkante sitz.

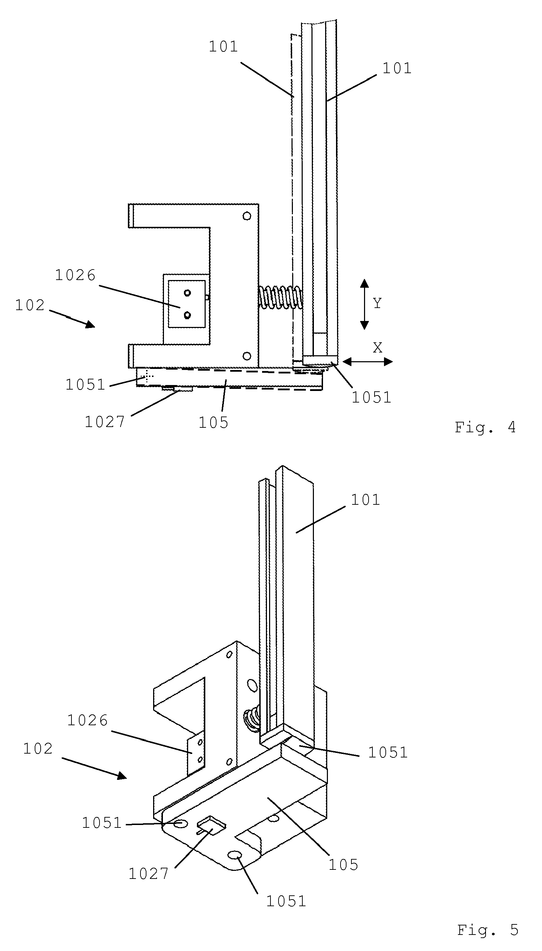

Figur 4 illustriert ein Detail aus Figur 3, wobei veranschaulicht wird, wie ein gegen die Führungsschiene wirkender Druckvektor zu einer Bewegung der Führungsschiene in einer X- und/oder Y-Bewegungskomponente führt, welche mittels des Kopplungselements 105 auf den Sensor 1027, bspw. Dehnmessstreifen, übertragen und von diesem in entsprechende Druckparameter umgewandelt wird. Dehnmessstreifen sind üblicherweise als Messeinrichtungen zur Erfassung von dehnenden Verformungen ausgebildet. Sie ändern bereits bei geringen Verformungen ihren elektrischen Widerstand und werden als Dehnungssensoren eingesetzt. Beim vorliegenden erfindungsgemässen System sind sie besonders geeignet, um Bewegungen von seitlich vom Bettinnern oder von oberhalb bzw. senkrecht auf die Holmen, Seile oder dergleichen ausgebildeten Seitensicherung ausgeübten Druck aufzunehmen.

Figur 5 illustriert eine perspektivische Ansicht der Anordnung von Führungsschiene 101, Kopplungselement 105 und Sensoren 1026,1027. Das Kopplungselement 105 ist bspw. mit Schrauben oder Bolzen am Bett befestigt. Das Kopplungselement 105 ist federnd bzw. beweglich gelagert. Es steht - ähnlich einer Lippe - etwas vor, so dass die Bewegungen der mit dem Kopplungselement gekoppelten Führungsschiene 101 übernommen werden und den Dehnmessstreifen in Dehnung versetzen. Das Kopplungselement selbst kann aus einem elastischen Material gefertigt sein. Vorzugsweise jedoch ist es aus Metall gebildet und derart gefertigt, dass die geforderte federnde Eigenschaft realisierbar ist.

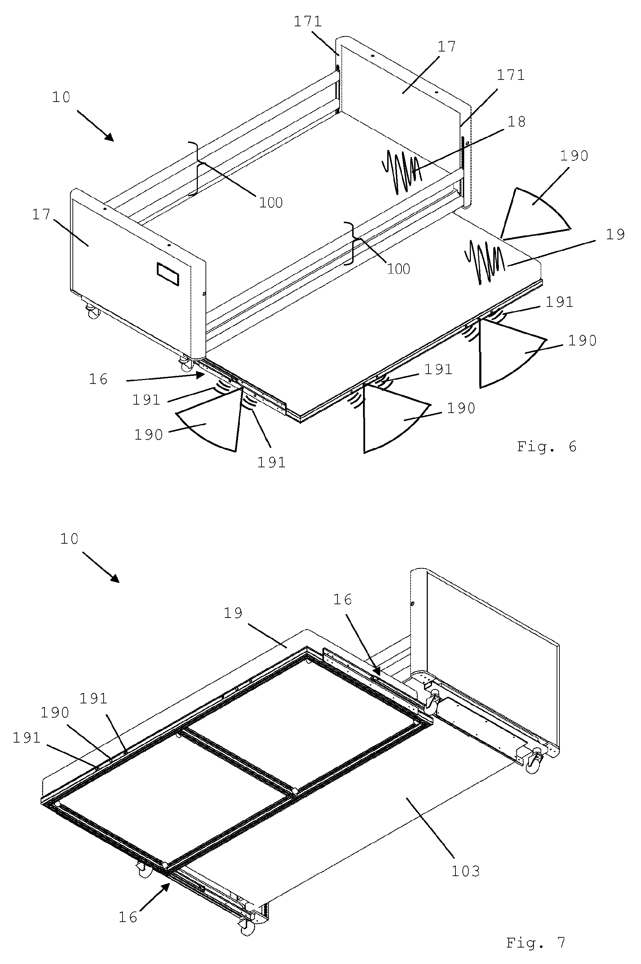

Figur 6 und Figur 7 illustrieren eine perspektivische Ansicht einer Ausführungsvariante des Personenüberwachungssystems, wobei das Bett 10 eine Lagereinrichtung 16 aufweist, mittels welcher ein Aufprallschutz 19, bspw. eine Matratze, verschiebbar am Bett gelagert ist. Die Lagereinrichtung 16 ist als verschiebbare Lagerebene ausgestaltet. Die Lagerebene kann als Stahlrahmen ausgeführt sein, welche schubladenartig ausziehbar ist. Der Rahmen weist Sensormittel 190 zum Erfassen von Bewegungen und zum Generieren von Sensorsignalen auf, mittels welchen in einem ausgezogenen Zustand, Bewegungen im Bereich des Aufprallschutzes 19 erfassbar sind und wobei basierend auf Sensorsignalen ein entsprechendes Überwachungssignal generierbar und mittels Interfacemodul 155 an das Empfangsmodul 157 übertragbar ist.

Die Lagereinrichtung 16 weist zur Wahrnehmbarmachung von Überwachungssignalen entsprechende optische und/oder akustische Warngeber 191 auf, mittels welchen beim Erfassen von Bewegungen im Bereich des Aufprallschutzes 19 entsprechende Warnsignale darstellbar sind. Die Warngeber sind als Leuchtmittel, bspw. LED, und/oder als Lautsprecher zum Darstellen entsprechender Warnsignale ausgebildet.

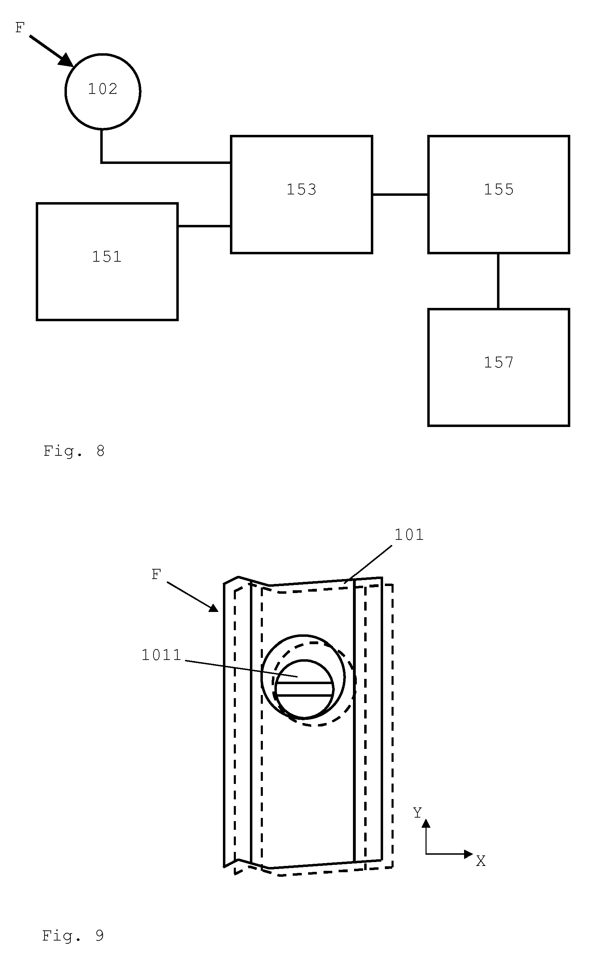

Figur 8 illustriert ein Blockschema des erfindungsgemässen Personenüberwachungssystems 1.

Jedem Druckvektor F sind die Druckparameter Druckdauer und/oder Druckrichtung und/oder Druckstärke zuordenbar und in einer Lookup-Table 151 speicherbar. Die Lookup-Table 151 wird vor in Betriebnahme des Personenüberwachungssystems 1 mit einer Vielzahl von Druckparametern vorkonfiguriert. Im Betrieb können mittels eines Eingabemoduls 158 des Personenüberwachungssystems 1 die Druckparameter an besondere Gegebenheiten angepasst, sprich modifiziert werden. Die Lookup-Table 151 kann auch als Speichermodul ausgebildet sein, bspw. einem lokalen Speicher - etwa einem USB Speicher, oder einem zentralen Speicher, bspw. einem Netzwerkspeicher. Den Druckparametern sind entsprechende Überwachungsmuster zugeordnet.

Das Interfacemodul ist 155 zum Übertragen von Überwachungssignalen als drahtgebundenes und/oder drahtloses Sende-Empfangsmodul zur Signal- bzw. Datenübertragung über LAN, WAN, Bluetooth etc. ausgebildet.

Das Empfangsmodul 157 ist zum Empfang und zum visuellen und/oder akustischen Darstellen von Überwachungssignalen ausgebildet. Es kann als Lautsprecher, Bildschirm, mobiles Anzeigegerät, bspw. ein Mobiltelefon, oder dergleichen ausgebildet sein.

Zur Erfassung des Druckvektors F ist mindestens ein druckempfindlicher Sensor, bspw. als Dehnmessstreifen, vorhanden, mittels welchem entsprechende Druckparameter als Druckdauer und/oder Druckrichtung und/oder Druckstärke generierbar und zwecks Vergleichs an ein Weichenmodul 153 übertragbar sind.

Das Weichenmodul 153 ist zum Vergleich von Druckparametern ausgebildet. In der erfindungsgemässen Ausführung werden elektrische Schaltzustände eines elektrischen Schalters 1026 und/oder elektrische Spannungen aus Spannungsbereichen von als Dehnmessstreifen ausgebildeten Sensoren 1027 erfasst und mit entsprechenden Druckparametern der Lookup-Table 151 verglichen. Das Weichenmodul 153 weist bestimmbare Vergleichskriterien auf, basierend auf welchen ein Vergleich von erfassten Druckparametern mit solchen aus der Lookup-Table als erfolgreich oder nicht erfolgreich durchgeführt gilt. Mittels Vergleichskriterien kann bspw. ein Bereich von Druckstärken festgelegt werden, welchem ein entsprechendes Überwachungsmuster zugeordnet wird. Das Weichenmodul kann in besonderen einfachen Fällen bspw. als Relaisschaltung kostengünstig realisiert werden.

Die Lookup-Table 151 umfasst zudem Überwachungsmuster, welche den jeweiligen Druckparametern zugeordnet sind. Ein Überwachungsmuster kann sein; dreimaliges kurzes seitliches Berühren oder Drücken der Seitensicherung 100 als bewusste Berührung, welche bedeutet, dass die bettlägerige Person um Hilfe ruft; fünf Sekunden ununterbrochenes seitliches Drücken gegen die Seitensicherung, welches bedeutet, dass die bettlägerige Person konkret Hunger oder Durst hat; unbewusstes ununterbrochenes seitliches Drücken mit einem Druck einer zu bestimmenden Anzahl N/mm² gegen die Seitensicherung wird als Überwachungsmuster in der Lookup-Table hinterlegt mit der Bedeutung, dass es sich um ein unbewusstes und unerwünschtes Verlagern der bettlägerigen Person handelt.

Die Module sind bspw. mittels einer speicherprogrammierbaren Steuerung realisiert. In einer kostensensitiven Ausführungsvariante sind als druckempfindliche Detektionsmittel bspw. elektrische Schalter oder Taster vorhanden. Jeder Bettpfosten weist bspw. Kopplungsmittel auf, um die auf die Holmen 1001 der Seitensicherung 100 ausgeführten Bewegungen auf die elektrischen Schalter zu übertragen. Die basierend auf den Bewegungen erfassten Schaltzustände werden in einem Zeitraster mittels der speicherprogrammierbaren Steuerung analysiert, mit den gespeicherten Überwachungsmustern verglichen und entsprechende Überwachungssignale mittels einem Interfacemodul des speicherprogrammierbaren Steuerung an ein Empfangsmodul 157, bspw. einen Lautsprecher oder einen Bildschirm übertragen und dargestellt.

Figur 9 illustriert eine Führungsschiene 101, welche ein Führungselement 1011 aufweist. Das Element wird gebildet durch eine Bohrung in der Schiene 101 und einem Befestigungsmittel 1011, bspw. einem Bolzen oder einer Schraube, mittels welcher die Schiene im nicht dargestellten Bettrahmen oder -pfosten beweglich gehalten ist. Die Differenz zwischen Durchmesser der Bohrung und Aussendurchmesser des Befestigungsmittels 1011 ist derart ausgebildet, dass bei Druck- bzw. Kraftwirkung F auf die Seitensicherung, welche in der Führungsschiene 101 gehalten ist, auf die Schiene 101 übertragen wird. Eine seitliche Kraftwirkung in Bewegungsrichtung X wird dabei in eine hier senkrechte oder vertikale Bewegungsrichtung Y umgewandelt und mittels der Führungsschiene auf das bspw. in Figur 4 dargestellte Kopplungselement 105 übertragen. Die Kraftwirkung wird dabei durch den Sensor 1027 erfasst und entsprechende Druckparameter werden als Sensorsignale generiert, insbesondere Druckstärke und/oder Druckrichtung und/oder Druckdauer.

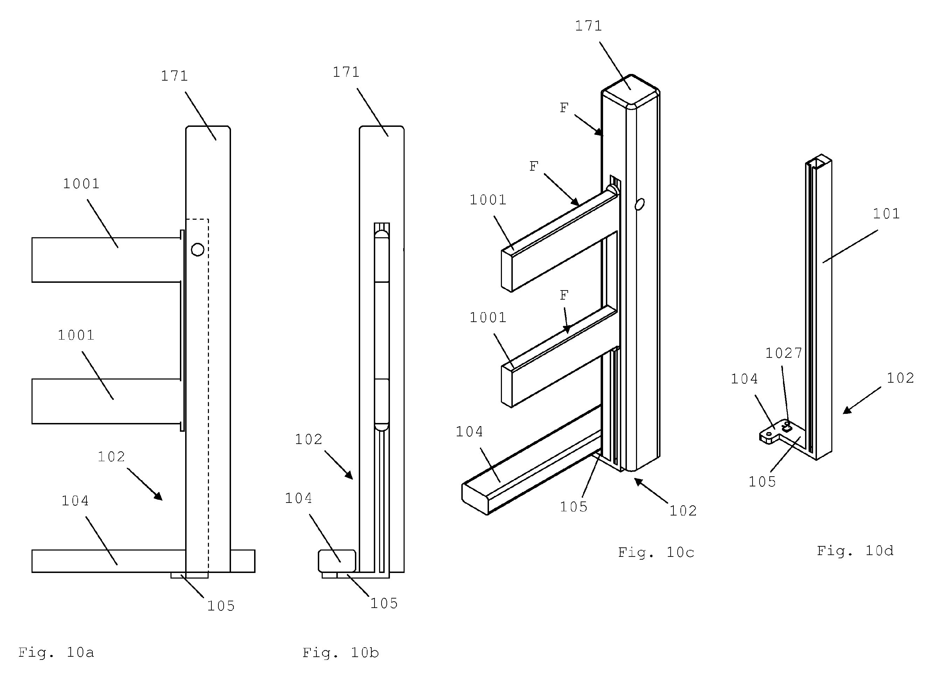

Figuren 10a, 10b sowie 10c illustrieren die beweglich gelagerte Seitensicherung 100 des erfindungsgemässen Personenüberwachungssystems 1 in einer abgeschnittenen schematischen Darstellung. Figur 10d zeigt in einem Detail der Figuren 10a bis 10c das Führungssystem bzw. die Führungsschiene 101, in welcher die Holmen 1001 zwecks Verstellen der Höhe geführt sind.

Die vorgeschlagene Befestigung der Seitensicherung 100, hier mit zwei Längs- bzw. Querholmen realisiert, an Bettpfosten oder -rahmen ist vor allem vorteilhaft bei Seitensicherungen bzw. Bettgittern, welche geteilt sind. Seitensicherungen also, welche nur entlang eines bestimmten oder entlang mehrerer Abschnitte der Bettseite angeordnet sind. Dies ist vorteilhaft bei Betten, welche einen meist starren Bettrahmen haben, bei welchen aber der Bettrost mehrteilig ausgebildet ist. Mittels mehrteiligen Bettrosten kann die Liegeposition verändert werden. So kann bspw. eine Sitzposition im Bett konfiguriert werden. Auch in diesen Fällen ist jeder Abschnitt der Seitensicherung 100 mit entsprechenden druckempfindlichen Detektionsmitteln gekoppelt.

Die Holmen 1001 können auf einer bestimmten Höhe arretiert werden. Sind die Holme arretiert, so wird jede Bewegung der bettlägerigen Person beim Berühren der Seitensicherung 100 auf die Führungsschiene 101 übertragen. Die Druckparameter des Druckvektor F werden mittels des druckempfindlichen Sensors 1026,1027, welcher bspw. als Dehnmessstreifen ausgebildet ist, erfasst und als Druckdauer und/oder Druckrichtung und/oder Druckstärke zum Zweck des Vergleichs mit Werten der Lookup-Table an das Weichenmodul übertragen.

Druckempfindliche Detektionsmittel 102 können auch als ohmsche, kapazitive, induktive oder optische Sensormittel realisiert sein. Zwecks Generierens von Alarmsignalen kann in besonderen Fällen ein elektrischer Schalter oder Taster als mit Öffner- oder Schliesser-Kontakt verwendet werden.

### Bezugszeichenlegende

- 1: Personenüberwachungssystem
- 10: Bett
- 100: Seitensicherung
- 1001: Holmen, Balken, Seil
- 101: Führungssystem, Kopplungssystem, Übertragungssystem
- 1011: Übertragungselement
- 102: druckempfindliche Detektionsmittel
- 1026: Schalter, Dehnmessstreifen
- 1027: Schalter, Dehnmessstreifen
- 103: Bettrahmen
- 104: Bettrostelement, Matratzenrost, Lattenrost
- 105: Kopplungselement, elastisches Element
- 1051: Kopplungselementbefestigung, Schwenkachse, Drehachse
- 151: Lookup-Table, Speichermodul
- 153: Weichenmodul
- 155: Interfacemodul
- 157: Empfangsmodul
- 158: Eingabemodul
- 16: Lagereinrichtung
- 17: Bettteil, Bettrahmenteil
- 18: Matratze
- 19: Aufprallschutz, Matratze
- 190: Sensormittel
- 191: Warngeber, Lautsprecher, Leuchte

- F: Druckvektor
- X: Bewegungskomponente
- Y: Bewegungskomponente

## Patentansprüche

1. Personenüberwachungssystem (1) zum Überwachen von Körperbewegungen einer Person in einem Bett (10), wobei das Bett eine Seitensicherung (100) an einer Seite des Betts umfasst, dass die Seitensicherung mit druckempfindlichen Detektionsmittel (102) gekoppelt ist, wobei Körperbewegungen der Person bei Berührung der Seitensicherung erfassbar und mittels eines Interfacemoduls (155) des Personenüberwachungssystems zum Übertragen eines entsprechenden Überwachungssignals an ein Empfangsmodul (157) übertragbar sind, **dadurch gekennzeichnet,**
**dass** die Seitensicherung (100) horizontal und vertikal beweglich gelagert ist, wobei mittels druckempfindlichem Detektionsmittel ein auf die Seitensicherung ausgeführter Druck als Druckvektor (F) erfassbar ist,
**dass** das Personenüberwachungssystem (1) eine Lookup-Table (151) zum Speichern von Druckparametern umfasst, wobei jedem Druckparameter entsprechende Überwachungsmuster zugeordnet sind,
**dass** mittels eines Weichenmoduls (153) des Personenüberwachungssystems (1) durch Vergleich von Druckparametern des erfassten Druckvektors mit Druckparametern der Lookup-Table (151) ein entsprechendes Überwachungsmuster aus der Lookup-Table auslesbar ist, basierend auf welchem ein entsprechendes Überwachungssignal generierbar ist und mittels Interfacemodul (155) an das Empfangsmodul (157) übertragbar ist.

2. Personenüberwachungssystem (1) gemäss Anspruch 1, **dadurch gekennzeichnet, dass** jedem Druckvektor entsprechende Druckparameter Druckdauer und/oder Druckrichtung und/oder Druckstärke zuordenbar und in der Lookup-Table (151) speicherbar sind, um mittels des Weichenmoduls (153) mit erfassbaren Druckvektoren (F) zu vergleichen, wobei basierend auf Vergleichskriterien entsprechende Überwachungsmuster aus der Lookup-Table auslesbar sind.

3. Personenüberwachungssystem (1) gemäss einem der Ansprüche 1 oder 2, **dadurch gekennzeichnet, dass** zur Erfassung des Druckvektors (F) mindestens ein druckempfindlicher Sensor, bspw. als Dehnmessstreifen, vorhanden ist, mittels welchem entsprechende Druckparameter als Druckdauer und/oder Druckrichtung und/oder Druckstärke generierbar und zwecks Vergleich an das Weichenmodul (153) übertragbar sind.

4. Personenüberwachungssystem (1) gemäss einem der Ansprüche 1 bis 3, **dadurch gekennzeichnet, dass** zur Erfassung des Druckvektors (F) mindestens ein elektrischer Schalter zum Erfassen von Druckdauer und/oder Druckrichtung und/oder Druckstärke vorhanden ist.

5. Personenüberwachungssystem (1) gemäss einem der Ansprüche 1 bis 4, **dadurch gekennzeichnet, dass** das Interfacemodul (155) zum Übertragen von Überwachungssignalen als drahtgebundenes und/oder drahtloses Sende-Empfangsmodul ausgebildet ist.

6. Personenüberwachungssystem (1) gemäss einem der Ansprüche 1 bis 5, **dadurch gekennzeichnet, dass** das Empfangsmodul (157) zum Empfang und zur visuellen und/oder akustischen Darstellen von Überwachungssignalen ausgebildet ist.

7. Personenüberwachungssystem (1) gemäss einem der Ansprüche 1 bis 6, **dadurch gekennzeichnet, dass** die Seitensicherung (100) in einem Führungssystem (101) höhenverstellbar und/oder schwenkbar gelagert ist, wobei das Führungssystem mit den Detektionsmitteln (102) gekoppelt ist.

8. Personenüberwachungssystem (1) gemäss einem der Ansprüche 1 bis 7, **dadurch gekennzeichnet, dass** die Seitensicherung (100) mehrteilig ausgebildet ist, wobei auf die bzw. jede horizontal und vertikal beweglich gelagerte Seitensicherung ausgeübter Druck mittels der druckempfindlichen Detektionsmittel (102) als Druckvektor erfassbar ist.

9. Personenüberwachungssystem (1) gemäss einem der Ansprüche 1 bis 8, **dadurch gekennzeichnet, dass** das Bett (10) eine Lagereinrichtung (16) aufweist, mittels welcher ein Aufprallschutz (19), bspw. eine Matratze, verschiebbar am Bett gelagert ist, dass die Lagereinrichtung (16) Sensormittel (190) zum Erfassen von Bewegungen und zum Generieren von Sensorsignalen aufweist, mittels welchen in einem ausgezogenen Zustand, Bewegungen im Bereich des Aufprallschutzes (19) erfassbar sind und wobei basierend auf Sensorsignalen ein entsprechendes Überwachungssignal generierbar und mittels Interfacemodul (155) an das Empfangsmodul (157) übertragbar ist.

10. Personenüberwachungssystem (1) gemäss Anspruch 9, **dadurch gekennzeichnet, dass** die Lagereinrichtung (16) zur Wahrnehmbarmachung von Überwachungssignalen entsprechende optische und/oder akustische Warngeber (191) aufweist, mittels welchen beim Erfassen von Bewegungen im Bereich des Aufprallschutzes (19) entsprechende Warnsignale darstellbar sind.

11. Personenüberwachungssystem (1) gemäss Anspruch 10, **dadurch gekennzeichnet, dass** die Warngeber als Leuchtmittel, bspw. LED, und/oder als Lautsprecher zum Darstellen entsprechender Warnsignale ausgebildet sind.

12. Verfahren zum Überwachen von Körperbewegungen einer Person in einem Bett (10), wobei Berührungen einer Seitensicherung (100) des Betts durch die Person mittels druckempfindlichen Detektionsmitteln (102) erfasst werden und ein entsprechendes Überwachungssignal generiert und an ein Empfangsmodul (157) des Personenüberwachungssystems (1) übertragen wird, **dadurch gekennzeichnet,**
**dass** die Seitensicherung (100) horizontal und vertikal beweglich gelagert ist, wobei mittels druckempfindlichem Detektionsmittel ein auf die Seitensicherung ausgeführter Druck als Druckvektor (F) erfasst und entsprechende Druckparameter generiert werden,
**dass** mittels eines Weichenmoduls (153) des Personenüberwachungssystems (1) die generierten Druckparameter mit in einer Lookup-Table (151) des Personenüberwachungssystems (1) speicherbaren Druckparametern verglichen werden,
**dass** den gespeicherten Druckparametern entsprechende Überwachungsmuster zugeordnet sind, wobei mittels bestimmbaren Vergleichskriterien des Weichenmoduls (153) beim Vergleich der Druckparameter ein entsprechendes Überwachungssignal generiert wird,
**dass** das Überwachungssignal mittels eines Interfacemoduls (155) des Personenüberwachungssystems (1) zum Übertragen von Signalen an das Empfangsmodul (157) übertragen wird.
